(19) Europäisches Patentamt / European Patent Office / Office européen des brevets

(11) **EP 3 929 935 A1**

(12) **EUROPEAN PATENT APPLICATION**
published in accordance with Art. 153(4) EPC

(43) Date of publication:
**29.12.2021 Bulletin 2021/52**

(51) Int Cl.:
*G16H 30/20* (2018.01)          *G16H 50/20* (2018.01)
*G06T 7/00* (2017.01)

(21) Application number: **19916480.7**

(22) Date of filing: **16.08.2019**

(86) International application number:
**PCT/CN2019/101156**

(87) International publication number:
**WO 2020/168694 (27.08.2020 Gazette 2020/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.02.2019 CN 201910132147**

(71) Applicant: **VR Doctor Medical Technology (Shenzhen) Co., Ltd.**
**Shenzhen, Guangdong, 518035 (CN)**

(72) Inventors:
• **LEE, Stewart Ping**
  **Shenzhen, Guangdong 518035 (CN)**
• **LEE, David Wei**
  **Shenzhen, Guangdong 518035 (CN)**

(74) Representative: **Dargiewicz, Joanna**
**JD&P Patent Attorneys**
**Joanna Dargiewicz & Partners**
**Ul. Mysliborska 93A/50**
**03-185 Warszawa (PL)**

(54) **VRDS 4D MEDICAL IMAGE-BASED AI PROCESSING METHOD AND PRODUCT FOR TUMORS**

(57)      Disclosed in embodiments of the present application are a method and a product for AI processing of tumor based on VRDS 4D medical images, which is applied to medical imaging apparatuses, and the method includes: determining a bitmap BMP data source according to a plurality of scanned images associated with a target organ of a target user; generating target medical image data according to the BMP data source; determining abnormal data in target medical image data; determining the attribute information of a tumor of the target user according to the abnormal data; performing 4D medical imaging according to the target medical image data, and outputting the attribute information of the tumor. The embodiment of this application is facilitated to improve the accuracy and efficiency of tumor recognition.

Fig. 2

## Description

## TECHNICAL FIELD

This application relates to the field of medical imaging apparatus, and in particular, to a method and a product for AI processing of tumor based on VRDS 4D medical images.

## BACKGROUND OF THE INVENTION

In current, doctors use technologies such as Computed Tomography (CT), Magnetic Resonance Imaging (MRI), Diffusion Tensor Imaging (DTI), Positron Emission Computed Tomography (PET) to acquire information such as the shape, location and topology of the pathological tissue. Doctors still use watching to read continuous two-dimensional slice scanned images, so as to perform judging and analyzing on the pathological tissue of the patients such as tumors. However, the two-dimensional slice scanned image can not present the spatial structure characteristics of tumors, which affects the diagnosis of the doctors on the diseases. With rapid development of medical imaging technology, people put forward new demands for medical imaging.

## SUMMARY

Embodiments of this application provide a method and a product for AI processing of tumor based on VRDS 4D medical images, in order to improve the accuracy and efficiency of the medical imaging apparatus in recognizing the tumors.

In a first aspect, embodiments of this application provide a method for AI processing of tumor based on VRDS 4D medical images, which is applied to medical imaging apparatus; and the method includes:

determining a bitmap BMP data source according to a plurality of scanned images associated with a target organ of a target user;

generating target medical image data according to the BMP data source, wherein the target medical image data includes at least a data set of the target organ and a data set of a blood vessel around the target organ, the data set of the blood vessel includes a data set of an artery and/or a data set of a vein, and the data of intersection positions of the artery and the vein are independent from each other, the data set of the target organ is a transfer function result of a cubic space between a surface of the target organ and a tissue structure inside the target organ, and the data set of the blood vessel is a transfer function result of a cubic space between a surface of the blood vessel and the tissue structure inside the blood vessel;

determining abnormal data in the target medical image data;

determining attribute information of a tumor of the target user according to the abnormal data;

performing a 4D medical imaging according to the target medical image data, and outputting the attribute information of the tumor.

In a second aspect, embodiments of this application provide an apparatus for processing tumor based on VRDS 4D medical images, the apparatus is applied to a medical imaging apparatus; the apparatus for processing tumor based on VRDS 4D medical image includes a processing unit and a communication unit, wherein,
the processing unit is configured to: introduce a bitmap BMP data source associated with a target organ of a target user into a preset VRDS medical network model to obtain a first medical image data, wherein the first medical image data includes the data set of the target organ and the data set of a blood vessel around the target organ, and the data set of the blood vessel includes fusion data of an intersection position between an artery and a vein; and compare the first medical image data with a pre-stored gray value template of the target tissue to determine normal data and abnormal data in the first medical image data, wherein the target tissue includes the target organ and the blood vessel; and determine attribute information of a tumor of the target user according to the abnormal data; and perform 4D medical imaging according to the normal data and abnormal data through the communication unit and output the attribute information of the tumor.

In a third aspect, embodiments of this application provide an medical imaging apparatus, the apparatus includes a processor, a memory, a communication interface, and one or more programs, wherein the above one or more programs are stored in the above memory and configured to be executed by the above processor, and the above programs include instructions for executing the steps in any method of the first aspect of the embodiment of this application.

In a fourth aspect, embodiments of this application provide a computer-readable storage medium, wherein the above computer-readable storage medium stores a computer program for electronic data exchange, wherein the above computer program causes a computer to execute some or all of the steps described in any methods of the first aspect of the embodiment of this application.

In a fifth aspect, embodiments of this application provide a computer program product, wherein the above computer program product includes a non-transitory computer-readable storage medium in which a computer program is stored, the above computer program is operable to cause the computer to execute some or all of the steps described in any methods of the first aspect of the embodiment of this application. The computer program product can be a software installation package.

As can be seen that in the embodiment of this application, first, the medical imaging apparatus determines a bitmap BMP data source according to a plurality of scanned images associated with a target organ of a target user; second, generates target medical image data according to the BMP data source; third, determines abnormal data in the target medical image data; fourth, determines the attribute information of a tumor of the target user according to the abnormal data; finally, performs 4D medical imaging according to the target medical image data, and outputs the attribute information of the tumor. Wherein the target medical image data includes at least a data set of the target organ and a data set of a blood vessel around the target organ, the data set of the blood vessel includes a data set of an artery and/or a data set of a vein, and data of intersection positions of the artery and the vein is independent from each other, the data set of the target organ is a transfer function result of a cubic space between a surface of the target organ and a tissue structure inside the target organ, and the data set of the blood vessel is a transfer function result of a cubic space between a surface of the blood vessel and a tissue structure inside the blood vessel. It can be seen that the medical imaging apparatus in this application can recognize and locate tumors and perform 4D medical imaging, which is facilitated to improve the accuracy and efficiency of tumor recognition.

## BRIEF DESCRIPTION OF THE DRAWINGS

In order to describe the technical solutions in the embodiments of this application or in the prior art more clearly, the following briefly describes the accompanying drawings required for describing the embodiments or the prior art, apparently, the accompanying drawings in the following description only show some embodiments of this application, and the ordinary skill person in the art may still derive other drawings from these accompanying drawings without creative efforts.

Fig. 1 is a schematic structural diagram of a medical image intelligent analysis and processing system based on VRDS 4D provided by an embodiment of this application;

Fig. 2 is a schematic flowchart of a method for AI processing of tumor based on VRDS 4D medical images provided by an embodiment of this application;

Fig. 3 is a schematic structural diagram of an medical imaging apparatus provided by an embodiment of this application;

Fig. 4 is a block diagram of functional units composition of an apparatus for processing tumor based on VRDS 4D medical images provided by an embodiment of this application.

## DETAILED DESCRIPTION OF PREFERRED EMBODIMENTS

In order to make person in the art better understand the solution of this application, the following clearly and completely describes the technical solutions in the embodiments of this application with reference to the accompanying drawings in the embodiments of this application, apparently, the described embodiments are only some but not all of the embodiments of this application. All other embodiments obtained by the ordinary skill person in the art based on the embodiments of this application without creative efforts shall fall within the protection scope of this application.

The terms "first", "second", etc. in the specification and claims of this application and the above drawings are used to distinguish different objects, but not to describe a specific order. Furthermore, that term "including" and "having" and any variations thereof are intended to cover non-exclusive inclusion. For example, a process, method, system, product or device including a series of steps or units is not limited to the listed steps or units, but optionally further includes steps or units not listed, or optionally further includes other steps or units inherent to these processes, methods, products or devices.

Reference to an "embodiment" herein means that a particular feature, structure or characteristic described in connection with an embodiment may be included in at least one embodiment of this application. The appearances of the phrase in various places in the specification are not necessarily all referring to the same embodiment, nor are separate or alternative embodiments mutually exclusive of other embodiments. It is explicitly and implicitly to be understood by the person skilled in the art that the described embodiment may be combined with other embodiments.

The medical imaging apparatuses related to the embodiments of this application refer to various instruments that use various different media as information carriers to reproduce the internal structure of the human body as images, and their image information has a corresponding relationship spatial and temporal distribution with the actual structure of the human body. "DICOM data" refers to the raw image file data collected by medical device and reflecting the internal structure features of human body, and can include information such as Computed Tomography (CT), Nuclear Magnetic Resonance (MRI), Diffusion Tensor Imaging (DTI), Positron Emission Computed Tomography (PET-CT), "image source" refers to Texture 2D/3D image volume data generated by parsing the raw DICOM data. "VRDS" refers to Virtual Reality Doctor system.

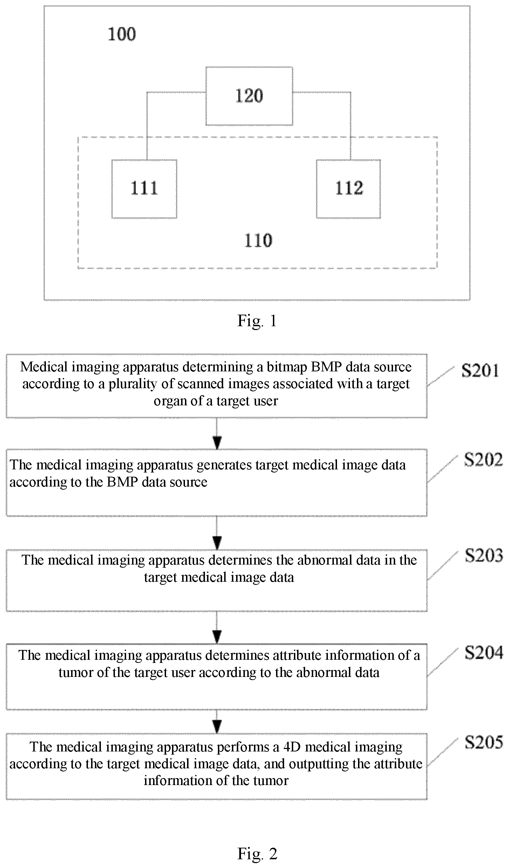

Referring to Fig. 1, Fig. 1 is a schematic structural diagram of a medical image intelligent analyzing and processing system 100 based on VRDS 4D provided by an embodiment of this application, the system 100 includes a medical imaging apparatus 110 and a network database 120, wherein the medical imaging apparatus 110 may include a local medical imaging apparatus 111 and/or a terminal medical imaging apparatus 112. The local medical imaging apparatus 111 or the terminal medical imaging apparatus 112 is configured to perform recognizing, positioning and four-dimensional 4D volume drawing on a human tumor region based on the raw DICOM data and the tumor recognition algorithm based on VRDS 4D medical image presented in the embodiment of this application, so as to achieve the 4D stereoscopic imaging effect (the four-dimensional medical image specifically refers to the medical image including the internal spatial structure features and the external spatial structure features of the displayed tissue, the internal spatial structure features refer to slice data inside the tissues are not lost, that is, medical imaging apparatuses can present the internal constructions of tissues such as target organs and blood vessels, and the external spatial structure characteristics refer to the environmental features between tissues, including spatial position characteristics (including intersection, spacing and fusion) between tissues, such as edge structure characteristics of the intersection position between kidney and artery, etc.). Compared with the terminal medical imaging apparatus 112, the local medical imaging apparatus 111 can further configured to edit the image source data to form the transfer function results of the four-dimensional human body image, which can include the transfer function results of the surface of the internal organs of the human body and the tissue structure inside the internal organs of the human body, as well as the transfer function results of the cube space, such as the number, coordinates, colors, transparency and other information of the cube editing boxes and arc editing arrays required by the transfer function. The network database 120 can be, for example, a cloud server, etc., the network database 120 is configured to store the image source generated by parsing the raw DICOM data and the transfer function result of the four-dimensional human body image edited by the local medical imaging apparatus 111, the image source can come from a plurality of local medical imaging apparatuses 111 to achieve the interactive diagnosis of a plurality of doctors.

When a user performs a specific image displaying through the above medical imaging apparatus 110, the user can choose a display and/or a head mounted displays set (HMDS) of virtual reality VR to display in combination with operation actions, which refer to the operation control of the four-dimensional human body image by the user through external intake device of the medical imaging apparatus, such as a mouse and a keyboard, so as to achieve human-computer interaction. The operation actions include at least one of the following: (1) changing the color and/or transparency of a specific organ/tissue, (2) positioning and scaling the view, (3) rotating the view to achieve multi-view 360-degree observation of the four-dimensional human image, (4) "entering" inside the organ of the human body to observe the internal construction, and the shearing effect rendering in real time, and (5) moving the view up and down.

The following describes the tumor recognition algorithm based on VRDS 4D medical image in detail.

Referring to Fig. 2, Fig. 2 is a schematic flowchart of a method for AI processing of tumor based on VRDS 4D medical images provided by an embodiment of this application, which is applied to medical imaging apparatus as described in Fig. 1. As shown in the Figure, the method for AI processing of tumor based on VRDS 4D medical images includes:

S201 , ,determining by a medical imaging apparatus, a bitmap BMP data source according to a plurality of scanned images associated with a target organ of a target user.

Wherein, the target organ can be, for example, a kidney. And the scanned image includes any one of the following: a CT image, an MRI image, a DTI image and a PET-CT image.

S202, the medical imaging apparatus generates target medical image data according to the BMP data source, wherein the target medical image data includes at least a data set of the target organ and a data set of a blood vessel around the target organ, the data set of the blood vessel includes a data set of an artery and/or a data set of a vein, and the data of intersection positions of the artery and the vein are independent from each other, the data set of the target organ is a transfer function result of a cubic space between a surface of the target organ and a tissue structure inside the target organ, and the data set of the blood vessel is a transfer function result of a cubic space between a surface of the blood vessel and the tissue structure inside the blood vessel.

In this possible example, the medical imaging apparatus generates the target medical image data according to the BMP data source, including: the medical imaging apparatus introduces the BMP data source into a preset VRDS medical network model to obtain a first medical image data, wherein the first medical image data includes the data set of the target organ and the data set of the blood vessel, and the data set of the blood vessel includes the fusion data of the intersection position of the artery and the vein; introduces the first medical image data into a preset cross blood vessel network model to obtain a second medical image data, wherein the second medical image data includes the data set of the target organ, the data set of the artery and the data set of the vein, and the first data in the data set of the artery and the second data in the data set of the vein are independent from each other; executes a first preset processing on the second medical image data to obtain target medical image data, wherein the first preset processing includes at least one of the following operations: 2D boundary optimization processing, 3D boundary optimization processing and data enhancement processing, and the target medical image data includes the data set of the target organ, the data set of the artery and the data set of the vein.

Wherein, the VRDS medical network model is provided with the transfer function of structural characteristics of target organs and the transfer function of structural characteristics of blood vessels, and the BMP data source obtains the first medical image data through the processing of the transfer function, and the cross blood vessel network model achieves the data separation of arteries and veins by the following operations: (1) extracting the fusion data of the intersection position; (2) for each fusion data, separating the fusion data based on a preset data separation algorithm to obtain artery boundary point data and vein boundary point data that are independent from each other; (3) integrating multiple artery boundary point data obtained after processing into first data and integrating multiple vein boundary point data obtained after processing into second data.

Wherein the 2D boundary optimization processing includes: acquiring low-resolution information and high-resolution information by sampling multiple times, wherein the low-resolution information can provide context semantic information of a segmentation target throughout the image, that is, features reflecting the relationship between the target and the environment, these features are used to judge the class of objects, and the high-resolution information is used to provide more fine features, such as gradients, for segmentation targets.

The 3D boundary optimization processing includes: 3D convolution, 3D maximum pooling and 3D upward convolution layer. the size of input data is a1, a2 and a3, the number of channels is c, and the size of the filter is f, that is, the dimension of the filter is f*f*f*c, and the number of filters is n, so the final output of 3 dimensional convolution is:

$$ (al\text{-}f\text{+}1) * (a2\text{-}f\text{+}1) * (a3\text{-}f\text{+}1) *n $$

which has an analysis path and a synthesis path. In the analysis path, each layer includes two convolution kernels of 3*3*3, each of which follows an activation function (Relu), and then there is a maximum pooling of 2*2*2 on each dimension to merge the two step lengths. In the synthesis path, each layer is composed of 2*2*2 upward convolution, and the step length on each dimension is 2, next, two 3*3*3 convolutions, and then Relu. Shortcut connections from equal resolution layers in the analysis path then provide the basic high-resolution features of the synthetic path. In the last layer, 1*1*1 convolution reduces the number of output channels.

Wherein, the data enhancement processing includes any one of the following: data enhancement based on arbitrary angle rotation, data enhancement based on histogram equalization, data enhancement based on white balance, data enhancement based on mirror operation, data enhancement based on random shearing and data enhancement based on simulating different illumination changes.

It can be seen that, in this example, the medical imaging apparatus can perform process on the BMP data source through the VRDS medical network model and the cross blood vessel network model and combine boundary optimization and data enhancement processing to obtain target image data, which solves the medical field problem that the traditional medical image cannot realize the whole separation of the segmented artery and vein, and improves the authenticity, comprehensiveness and refinement of the medical image display.

In this possible example, the medical imaging apparatus determines the bitmap BMP data source according to a plurality of scanned images associated with the target organ of the target user, including: the medical imaging apparatus acquires a plurality of scanned images collected by a medical device and reflecting internal structure features of human body of the target user; screens at least one scanned image including the target organ from the plurality of scanned images, and takes the at least one scanned image as medical digital imaging and communication DICOM data of the target user; parses the DICOM data to generate a image source of the target user, wherein the image source includes Texture 2D/3D image volume data; executes a second preset process for the image source to obtain the BMP data source, wherein the second preset process includes at least one of the following operations: VRDS limited contrast adaptive histogram equalization, mixed partial differential denoising and VRDS AI elastic deformation processing.

Wherein, the VRDS limited contrast adaptive histogram equalization includes: regional noise ratio limiting and global contrast limiting; the local histogram of the image source is divided into a plurality of partitions, for each partition, the slope of the transform function is determined according to the slope of the cumulative histogram of the neighborhood of the partition, and the contrast amplification degree around the pixel value of the partition is determined according to the slope of the transform function, then, according to the contrast amplification degree, the limit clipping process is carried out to generate the distribution of effective histograms and the value of effective available neighborhood size, and these clipped partial histograms are uniformly distributed to other areas of the histogram.

Wherein, the mixed partial differential denoising includes: different from Gaussian low-pass filtering (which weakens the high-frequency components of the image indiscriminately and blurs the edges of the image while denoising), the isophotes (including edges) formed by objects in the natural image should be smooth and unhindered curves, that is, the absolute value of curvature of these isophotes should be small enough, and when the image is polluted by noise, the local gray value of the image will fluctuate randomly, and it leads to the irregular oscillation of the isophote and forms the isophote with large local curvature, and according to this principle, a mixed partial differential denoising model is designed, which can protect the image edge and avoid the step effect in the smoothing process by VRDS AI curvature driving and VRDS AI high-order hybrid denoising.

Wherein, the VRDS AI elastic deformation processing includes: superimposing positive and negative random distances on the original lattice to form a difference position matrix, and then forming a new lattice at the gray level of each difference position, so as to achieve the internal distortion of the image, and further performing rotation, distortion and translation operations etc. on the image.

It can be seen that in this example, the medical imaging apparatus obtains the BMP data source by processing the raw scanned image data, which improves the information amount of the raw data and increases the depth dimension information, and finally obtains the data meeting the display requirements of the 4D medical image.

S203, the medical imaging apparatus determines of the abnormal data in the target medical image data;

in specific implementation, the implementation way of the medical imaging apparatus determining of abnormal data in the target medical image data may be: comparing the target medical image data with a pre-stored gray value template of the target tissue to obtain abnormal data with an unmatched comparison result, wherein the target tissue includes the target organ and the blood vessel.

S204, the medical imaging apparatus determines attribute information of a tumor of the target user according to the abnormal data.

Wherein, the attribute information of the tumor includes any one of the following: type attribute, size attribute and position attribute.

In this possible example, the attribute information includes a location attribute; the medical imaging apparatus determines the attribute information of the tumor of the target user according to the abnormal data including: the medical imaging apparatus detects a tissue associated with the abnormal data; determines that the location attribute of the tumor is an internal tumor if detecting that the tissue associated with the abnormal data only includes the target organ; and determines that the location attribute of the tumor is an external tumor if detecting that the tissue associated with the abnormal data includes the target organ and the blood vessel.

Wherein, taking the kidney as an example, if the tissue associated with the current abnormal data only includes the kidney, that is, the abnormal data is in the spatial range of the kidney, then the tumor is determined to be the tumor inside the kidney.

It can be seen that, in this example, the medical imaging apparatus can accurately determine the location information of the tumor through the association analysis of the tissues described in the data, and because of the high accuracy of data comparison, the difference of doctors' eye observation is avoided, and the accuracy and efficiency of tumor location recognition are improved.

In this possible example, the attribute information includes a type attribute; the medical imaging apparatus determines the attribute information of the tumor of the target user according to the abnormal data including: the medical imaging apparatus determines boundary abnormal data in the abnormal data; determines the boundary characteristics of the tumor according to the boundary abnormality data; determines that the type attribute of the tumor of the target user is benign tumor if detecting that the boundary characteristics are non-smooth continuous characteristics; and determines that the type attribute of the tumor of the target user is malignant tumor if detecting that the boundary characteristics are non-smooth continuous characteristics.

Wherein, tumor is a new organism formed by abnormal proliferation and differentiation caused by the loss of normal regulation on the growth of the cells of local tissue at the gene level under the action of various tumorigenic factors. Benign tumor refers to a tumor that has no ability of invasion and metastasis. Benign tumors often have capsule, clear boundary and present smooth continuous characteristic. Malignant tumor is a kind of cellular disease, which is characterized by sudden mutation of genetic gene, resulting in persistent abnormal hyperproliferation of cells to form a lump, because of its invasion to surrounding tissues and transfer to other organs, the boundary is usually unclear, showing non-smooth continuous characteristics.

It can be seen that in this example, the medical imaging apparatus determines the boundary characteristics of tumors by analyzing the boundary data of tumors, and accurately determines the types of tumors according to the boundary characteristics, which is facilitated to improve the accuracy and efficiency of tumor type recognition.

In this possible example, the attribute information includes a size attribute; the medical imaging apparatus determines the attribute information of the tumor of the target user according to the abnormal data including: the medical imaging apparatus determines spatial coordinate information of each abnormal data in the constant data; and determines the size attribute of the tumor of the target user according to the spatial coordinate information of each abnormal data.

Wherein, by analyzing the size of tumor periodically, one can get the growth characteristics of the tumor accurately, thereby providing accurate information support for the treatment of the tumors.

It can be seen that in this example, the medical imaging apparatus can accurately calculate the size of the tumor based on the spatial coordinate information, and the tumor size can accurately reflect the growth state of the tumor, thus providing accurate information support for the treatment of the tumor.

S205, the medical imaging apparatus performs a 4D medical imaging according to the target medical image data, and outputs the attribute information of the tumor.

Wherein, the 4D medical imaging refers to presenting a the four-dimensional medical image.

In this possible example, the medical imaging apparatus preforms the 4D medical imaging according to the target medical image data, including: the medical imaging apparatus screens enhanced data with a quality score greater than a preset score from the target medical image data as VRDS 4D imaging data.

Wherein, the quality score can be comprehensively evaluated from the following dimensions: average gradient, information entropy, visual information fidelity, peak signal-to-noise ratio (PSNR), structural similarity (SSIM), mean square error (MSE), etc, specific reference can be made to common image quality score algorithms in the image field, which is not described details for brevity.

It can be seen that, in this example, the medical imaging apparatus further performs data screening through the quality score, thus improving the imaging effect.

In this possible example, the specific implementation of the medical imaging apparatus outputting the attribute information of the tumor may be: the medical imaging apparatus preforms outputting the attribute information at a preset position of a display screen displaying an image of the tumor when a selection operation for a position of the tumor is detected.

Wherein, the selection operation can be specifically selected by the user through the mouse, or through a head-mounted VR device to control the viewing angle for selection, etc, which is not uniquely limited here, and it can be seen that the triggering process of the display of the attribute information is convenient and efficient.

As can be seen that in the embodiment of this application, first, the medical imaging apparatus determines a bitmap BMP data source according to a plurality of scanned images associated with a target organ of a target user; second, generates target medical image data according to the BMP data source; third, determines abnormal data in the target medical image data; fourth, determines the attribute information of a tumor of the target user according to the abnormal data; finally, performs 4D medical imaging according to the target medical image data, and outputs the attribute information of the tumor. Wherein the target medical image data includes at least a data set of the target organ and a data set of a blood vessel around the target organ, the data set of the blood vessel includes a data set of an artery and/or a data set of a vein, and data of intersection positions of the artery and the vein is independent from each other, the data set of the target organ is a transfer function result of a cubic space between a surface of the target organ and a tissue structure inside the target organ, and the data set of the blood vessel is a transfer function result of a cubic space between a surface of the blood vessel and a tissue structure inside the blood vessel. It can be seen that the medical imaging apparatus in this application can recognize and locate tumors and perform 4D medical imaging, which is facilitated to improve the accuracy and efficiency of tumor recognition.

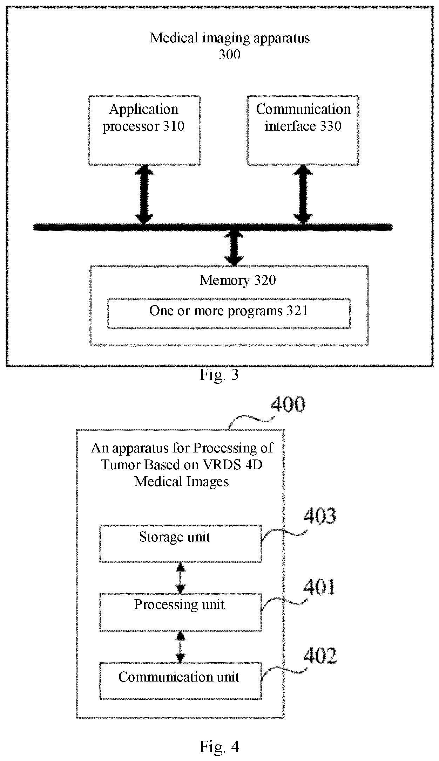

Consistent with the embodiments shown in Fig. 2, Fig. 3 and Fig. 4, referring to Fig. 3, Fig. 3 is a schematic structural diagram of a medical imaging apparatus 300 provided by an embodiment of this application, as shown in the Figure, the medical imaging apparatus 300 includes a processor 310, a memory 320, a communication interface 330 and one or more programs 321, wherein the one or more programs 321 are stored in the above memory 320 and configured to be executed by the above processor 310, and the one or more programs 321 include instructions for executing the following steps: determining a bitmap BMP data source according to a plurality of scanned images associated with a target organ of a target user; generating target medical image data according to the BMP data source, wherein the target medical image data includes at least a data set of the target organ and a data set of a blood vessel around the target organ, the data set of the blood vessel includes a data set of an artery and/or a data set of a vein, and data of intersection positions of the artery and the vein is independent from each other, the data set of the target organ is a transfer function result of a cubic space between a surface of the target organ and a tissue structure inside the target organ, and the data set of the blood vessel is a transfer function result of a cubic space between a surface of the blood vessel and a tissue structure inside the blood vessel; and determining abnormal data in the target medical image data; and determining attribute information of a tumor of the target user according to the abnormal data; and performing 4D medical imaging according to the target medical image data and outputting the attribute information of the tumor.

As can be seen that in the embodiment of this application, first, the medical imaging apparatus determines a bitmap BMP data source according to a plurality of scanned images associated with a target organ of a target user; second, generates target medical image data according to the BMP data source; third, determines abnormal data in the target medical image data; fourth, determines the attribute information of a tumor of the target user according to the abnormal data; finally, performs 4D medical imaging according to the target medical image data, and outputs the attribute information of the tumor. Wherein the target medical image data includes at least a data set of the target organ and a data set of a blood vessel around the target organ, the data set of the blood vessel includes a data set of an artery and/or a data set of a vein, and data of intersection positions of the artery and the vein is independent from each other, the data set of the target organ is a transfer function result of a cubic space between a surface of the target organ and a tissue structure inside the target organ, and the data set of the blood vessel is a transfer function result of a cubic space between a surface of the blood vessel and a tissue structure inside the blood vessel. It can be seen that the medical imaging apparatus in this application can recognize and locate tumors and perform 4D medical imaging, which is facilitated to improve the accuracy and efficiency of tumor recognition.

In one possible example, in the aspect of the attribute information includes a location attribute; the determining of the attribute information of the tumor of the target user according to the abnormal data, the instructions in the program are specifically configured to perform the following operations: detecting a tissue associated with the abnormal data; and determining that the location attribute of the tumor is an internal tumor if detecting that the tissue associated with the abnormal data only includes the target organ; and determining that the location attribute of the tumor is an external tumor if detecting that the tissue associated with the abnormal data includes the target organ and the blood vessel.

In one possible example, the attribute information includes a type attribute; in the aspect of the determining of the attribute information of the tumor of the target user according to the abnormal data, the instructions in the program are specifically configured to perform the following operations: determining boundary abnormal data in the abnormal data; and determining the boundary characteristics of the tumor according to the boundary abnormality data; and determining that the type attribute of the tumor of the target user is benign tumor if determining that the boundary characteristics are non-smooth continuous characteristics; and determining that the type attribute of the tumor of the target user is malignant tumor if detecting that the boundary characteristics are non-smooth continuous characteristics.

In one possible example, the attribute information includes a size attribute; in the aspect of the determining of the attribute information of the tumor of the target user according to the abnormal data, the instructions in the program are specifically configured to perform the following operations: determining spatial coordinate information of each abnormal data in the constant data; and determining the size attribute of the tumor of the target user according to the spatial coordinate information of each abnormal data.

In one possible example, in the aspect of the outputting of the attribute information of the tumor, the instructions in the program are specifically configured to perform the following operations: outputting the attribute information at a preset position of a display screen displaying an image of the tumor when a selection operation for a position of the tumor is detected.

In one possible example, in the aspect of the generating of the target medical image data according to the BMP data source, the instructions in the program are specifically configured to perform the following operations: introducing the BMP data source into a preset VRDS medical network model to obtain a first medical image data, wherein the first medical image data includes the data set of the target organ and the data set of the blood vessel, and the data set of the blood vessel includes the fusion data of the intersection position of the artery and the vein; and introducing the first medical image data into a preset cross blood vessel network model to obtain a second medical image data, wherein the second medical image data includes the data set of the target organ, the data set of the artery and the data set of the vein, and the first data in the data set of the artery and the second data in the data set of the vein are independent from each other; and executing a first preset processing on the second medical image data to obtain target medical image data, wherein the first preset processing includes at least one of the following operations: 2D boundary optimization processing, 3D boundary optimization processing and data enhancement processing, and the target medical image data includes the data set of the target organ, the data set of the artery and the data set of the vein.

In one possible example, in the aspect of the performing of the 4D medical imaging according to the target medical image data, the instructions in the program are specifically configured to perform the following operation: the medical imaging apparatus screens enhanced data with a quality score greater than a preset score from the target medical image data as VRDS 4D imaging data.

The above mainly introduces the scheme of the embodiment of this application from the perspective of the execution process on the method side. It can be understood that in order to achieve the above functions, the medical imaging apparatus includes corresponding hardware structures and/or software modules for performing various functions. It should be easy for person of skill in the art aware that, in combination with the units and algorithmic steps of the examples described in the embodiments provided herein, this application can be implemented in the form of hardware or a combination of hardware and computer software. Whether the functions are performed by hardware or computer software driving hardware depends on particular applications and design constraint conditions of the technical solutions. A person skilled in the art may use different methods to implement the described functions for each particular application, but it should not be considered that the implementation goes beyond the scope of this application.

The embodiment of this application can divide the medical imaging apparatus into functional units according to the above method example, for example, individual functional unit can be divided corresponding to individual function, or two or more functions can be integrated into one processing unit. The integrated units can be implemented in the form of hardware, and can also be implemented in the form of a software functional unit. It should be noted that the division of units in the embodiment of this application is schematic, which is only a logical function division, and there may be another division mode in actual implementation.

Fig. 4 is a block diagram of functional units composition of an apparatus 400 for processing tumor based on VRDS 4D medical images involved in the embodiment of this application. The apparatus 400 for processing tumor based on VRDS 4D medical images is applied to a medical imaging apparatus, the apparatus 400 for processing tumor based on VRDS 4D medical image includes a processing unit 401 and a communication unit 402.

The processing unit 401 is configured to: introduce a bitmap BMP data source associated with a target organ of a target user into a preset VRDS medical network model to obtain a first medical image data, wherein the first medical image data includes the data set of the target organ and the data set of a blood vessel around the target organ, and the data set of the blood vessel includes fusion data of an intersection position between an artery and a vein; compare the first medical image data with a pre-stored gray value template of the target tissue to determine normal data and abnormal data in the first medical image data, wherein the target tissue includes the target organ and the blood vessel; determine attribute information of a tumor of the target user according to the abnormal data; and perform 4D medical imaging according to the normal data and abnormal data through the communication unit 402 and output the attribute information of the tumor.

The apparatus 400 for processing tumor based on VRDS 4D medical images further includes a storage unit 403, wherein the processing unit 401 can be a processor, the communication unit 402 can be a transceiver, and the storage unit can be a memory.

As can be seen that in the embodiment of this application, first, the medical imaging apparatus determines a bitmap BMP data source according to a plurality of scanned images associated with a target organ of a target user; second, generates target medical image data according to the BMP data source; third, determines abnormal data in the target medical image data; fourth, determines the attribute information of a tumor of the target user according to the abnormal data; finally, performs 4D medical imaging according to the target medical image data, and outputs the attribute information of the tumor. Wherein the target medical image data includes at least a data set of the target organ and a data set of a blood vessel around the target organ, the data set of the blood vessel includes a data set of an artery and/or a data set of a vein, and data of intersection positions of the artery and the vein is independent from each other, the data set of the target organ is a transfer function result of a cubic space between a surface of the target organ and a tissue structure inside the target organ, and the data set of the blood vessel is a transfer function result of a cubic space between a surface of the blood vessel and a tissue structure inside the blood vessel. It can be seen that the medical imaging apparatus in this application can recognize and locate tumors and perform 4D medical imaging, which is facilitated to improve the accuracy and efficiency of tumor recognition.

In one possible example, the attribute information includes a location attribute; in the aspect of the determining of the attribute information of the tumor of the target user according to the abnormal data, the processing unit 401 are specifically configured to: detect a tissue associated with the abnormal data; and determine that the location attribute of the tumor is an internal tumor if detecting that the tissue associated with the abnormal data only includes the target organ; and determine that the location attribute of the tumor is an external tumor if detecting that the tissue associated with the abnormal data includes the target organ and the blood vessel.

In one possible example, the attribute information includes a type attribute; in the aspect of the determining of the attribute information of the tumor of the target user according to the abnormal data, the processing unit 401 are specifically configured to: determine boundary abnormal data in the abnormal data; and determine the boundary characteristics of the tumor according to the boundary abnormality data; and determine that the type attribute of the tumor of the target user is benign tumor if determining that the boundary characteristics are non-smooth continuous characteristics; and determine that the type attribute of the tumor of the target user is malignant tumor if detecting that the boundary characteristics are non-smooth continuous characteristics.

In one possible example, the attribute information includes a size attribute; in the aspect of the determining of the attribute information of the tumor of the target user according to the abnormal data, the processing unit 401 are specifically configured to: determine spatial coordinate information of each abnormal data in the constant data; and determine the size attribute of the tumor of the target user according to the spatial coordinate information of each abnormal data.

In one possible example, in the aspect of the outputting of the attribute information of the tumor, the processing unit 401 specifically configured to: output the attribute information at a preset position of a display screen displaying an image of the tumor when a selection operation for a position of the tumor is detected.

In one possible example, in the aspect of the generating of the target medical image data according to the BMP data source, the processing unit 401 are specifically configured to: introduce the BMP data source into a preset VRDS medical network model to obtain a first medical image data, wherein the first medical image data includes the data set of the target organ and the data set of the blood vessel, and the data set of the blood vessel includes the fusion data of the intersection position of the artery and the vein; and introduce the first medical image data into a preset cross blood vessel network model to obtain a second medical image data, wherein the second medical image data includes the data set of the target organ, the data set of the artery and the data set of the vein, and the first data in the data set of the artery and the second data in the data set of the vein are independent from each other; and execute a first preset processing on the second medical image data to obtain target medical image data, wherein the first preset processing includes at least one of the following operations: 2D boundary optimization processing, 3D boundary optimization processing and data enhancement processing, and the target medical image data includes the data set of the target organ, the data set of the artery and the data set of the vein.

In one possible example, in the aspect of the performing of the 4D medical imaging according to the target medical image data, the processing unit 401 are specifically configured to: screen enhanced data with a quality score greater than a preset score from the target medical image data as VRDS 4D imaging data.

Embodiments of this application further provide a computer storage medium, wherein the computer storage medium stores a computer program for electronic data exchange, the computer program causes a computer to execute part or all of the steps of any method as recorded in the above method embodiment, and the above computer includes a medical imaging apparatus.

Embodiments of this application further provide a computer program product, the above computer program product includes a non-transitory computer-readable storage medium in which a computer program is stored, the above computer program is operable to cause a computer to execute part or all of the steps of any method as recorded in the above method embodiments. The computer program product can be a software installation package, and the above computer includes a medical imaging apparatus.

It should be noted that, for brevity of description, the foregoing method embodiments are described as a series of movement combinations. However, a person skilled in the art should learn that this application is not limited by the movement sequence, because according to this application, some steps can be performed in other order or simultaneously. Secondly, it also should be known by those skilled in the art that the embodiments described in the specification are preferred embodiments and the involved actions and modules are not the must of the present application necessarily.

In the above embodiments, the descriptions of individual embodiment have their own emphasis, for those parts that are not detailed in one embodiment, please refer to the relevant descriptions of other embodiments.

In the several embodiments provided in this application, it should be understood that the disclosed apparatus may be implemented in other manners. For example, the described apparatus embodiment is merely an example. For example, the above unit division is merely logical function division and may be other division in actual implementation. For example, a plurality of units or components may be combined or integrated into another system, or some features may be ignored or not performed. In addition, the displayed or discussed mutual couplings or direct couplings or communication connections may be implemented by using some interfaces. The indirect couplings or communication connections between the apparatuses or units may be implemented in electronic, or other forms.

The above units described as separate parts may or may not be physically separate, and parts displayed as units may or may not be physical units, may be located in one position, or may be distributed on a plurality of network units. It can select some or all of units to achieve the objective of the solution of the present embodiment based on actual requirements.

In addition, functional units in the embodiments of this application may be integrated into one processing unit, or each of the units may exist alone physically, or two or more units are integrated into one unit. The integrated units can be implemented in the form of hardware, and can also be implemented in the form of a software functional unit.

When the above integrated units are implemented in the form of a software functional unit and sold or used as an independent product, the functions may be stored in a computer-readable memory. Based on such an understanding, the technical solutions of this application essentially, or the part contributing to the prior art, all or some of the technical solutions may be implemented in a form of a software product. The computer software product is stored in a memory, and includes several instructions for instructing a computer device (which may be a personal computer, a server, or a network device) to perform all or some of the steps of the above methods described in the embodiments of this application. The foregoing memory includes: any medium that can store program code, such as a USB flash disk, a read-only memory (ROM), a random access memory (RAM), a removable hard disk, a magnetic disk, or an optical disc.

Those skilled in the art may understand that all or some of the steps in various methods of the above embodiments can be completed by instructing related hardware through programs, which can be stored in a computer-readable memory, which can include: flash disks, a read-only memory (ROM), a random access memory (RAM), disks or optical disks, etc.

The embodiments of this application are described in detail above, and the principles and implementation of this application are explained by specific examples. The above embodiments are only used to help understand the method and its core ideas of this application; at the same time, according to the idea of this application, there will be some changes in the specific implementation and application scope for a person of skill in the art. As above, the contents of this specification should not be construed as limitations of this application.

**Claims**

**1.** A method for AI processing of tumor based on Virtual Reality Doctor system (VRDS) 4D medical images, **characterized in that**, the method is applied to medical imaging apparatuses; and the method comprises:

determining a bitmap (BMP) data source according to a plurality of scanned images associated with a target organ of a target user; generating target medical image data according to the BMP data source, wherein the target medical image data comprises at least a data set of the target organ and a data set of a blood vessel around the target organ, the data set of the blood vessel comprises a data set of an artery and/or a data set of a vein, and the data of intersection positions of the artery and the vein are independent from each other, the data set of the target organ is a transfer function result of a cubic space between a surface of the target organ and a tissue structure inside the target organ, and the data set of the blood vessel is a transfer function result of a cubic space between a surface of the blood vessel and the tissue structure inside the blood vessel; determining abnormal data in the target medical image data; determining attribute information of a tumor of the target user according to the abnormal data; performing a 4D medical imaging according to the target medical image data, and outputting the attribute information of the tumor.

2. The method according to claim 1, **characterized in that** the attribute information comprises a location attribute; the determining of the attribute information of the tumor of the target user according to the abnormal data comprises:

detecting a tissue associated with the abnormal data; if detecting that the tissue associated with the abnormal data only comprises the target organ, determining that the location attribute of the tumor is an internal tumor; if detecting that the tissue associated with the abnormal data comprises the target organ and the blood vessel, determining that the location attribute of the tumor is an external tumor.

3. The method according to claim 1, **characterized in that** the attribute information comprises a type attribute; the determining the attribute information of the tumor of the target user according to the abnormal data comprises:

determining boundary abnormal data in the abnormal data; determining the boundary characteristics of the tumor according to the boundary abnormality data; if detecting that the boundary characteristics are smooth continuous characteristics, determining that the type attribute of the tumor of the target user is benign tumor; if detecting that the boundary characteristics are non-smooth continuous characteristics, determining that the type attribute of the tumor of the target user is malignant tumor.

4. The method according to claim 1, **characterized in that** the attribute information comprises a size attribute; the determining of the attribute information of the tumor of the target user according to the abnormal data comprises:

determining spatial coordinate information of each abnormal data in the constant data; determining the size attribute of the tumor of the target user according to the spatial coordinate information of each abnormal data.

5. The method according to any one of claims 1 to 4, **characterized in that** the outputting of the attribute information of the tumor comprises: outputting the attribute information at a preset position of a display screen displaying an image of the tumor when a selection operation for a position of the tumor is detected.

6. The method according to any one of claims 1-5, **characterized in that** the generating target medical image data according to the BMP data source comprises:

introducing the BMP data source into a preset VRDS medical network model to obtain a first medical image data, wherein the first medical image data comprises the data set of the target organ and the data set of the blood vessel, and the data set of the blood vessel comprises fusion data of an intersection position between an artery and a vein; introducing the first medical image data into a preset cross blood vessel network model to obtain a second medical image data, wherein the second medical image data comprises the data set of the target organ, the data set of the artery and the data set of the vein, and first data in the data set of the artery and second data in the data set of the vein are independent from each other; the first data is a data associated with the intersection position, and the second data is a data associated with the intersection position; executing a first preset processing on the second medical image data to obtain target medical image data, wherein the first preset processing comprises at least one of the following operations: 2D boundary optimization processing, 3D boundary optimization processing and data enhancement processing, and the target medical image data comprises the data set of the target organ, the data set of the artery and the data set of the vein.

7. The method according to claim 6, **characterized in that** the performing of the 4D medical imaging according to the target medical image data comprises: screening enhanced data with a quality score greater than a preset score from the target medical image data as VRDS 4D imaging data.

8. The method according to claim 1, **characterized in that** the determining of the bitmap BMP data source according to a plurality of scanned images associated with the target organ of the target user comprises:

   acquiring a plurality of scanned images collected by a medical device and reflecting internal structural features of human body of the target user; screening at least one scanned image including the target organ from the plurality of scanned images, and taking the at least one scanned image as medical digital imaging and communication DICOM data of the target user; parsing the DICOM data to generate a image source of the target user, wherein the image source comprises Texture 2D/3D image volume data;
   executing a second preset processing for the image source to obtain the BMP data source, wherein the second preset processing comprises at least one of the following operations: VRDS limited contrast adaptive histogram equalization, mixed partial differential denoising and VRDS AI elastic deformation processing.

9. The method according to claim 1, **characterized in that** the determining of the abnormal data in the target medical image data comprises:
   comparing the target medical image data with a pre-stored gray value template of the target tissue to obtain abnormal data with an unmatched comparison result, wherein the target tissue comprises the target organ and the blood vessel.

10. An apparatus for processing tumor based on VRDS 4D medical images, **characterized in that** the apparatus is applied to a medical imaging apparatus; the apparatus for processing tumor based on VRDS 4D medical image comprises a processing unit and a communication unit, wherein
    the processing unit is configured to: introduce a bitmap BMP data source associated with a target organ of a target user into a preset VRDS medical network model to obtain a first medical image data, wherein the first medical image data comprises the data set of the target organ and the data set of a blood vessel around the target organ, and the data set of the blood vessel comprises fusion data of an intersection position between an artery and a vein; compare the first medical image data with a pre-stored gray value template of the target tissue to determine normal data and abnormal data in the first medical image data, wherein the target tissue comprises the target organ and the blood vessel; determine attribute information of a tumor of the target user according to the abnormal data; and perform 4D medical imaging according to the normal data and abnormal data through the communication unit and output the attribute information of the tumor.

11. The apparatus according to claim 10, **characterized in that** the attribute information comprises a location attribute; in the aspect of the determining of the attribute information of the tumor of the target user according to the abnormal data, the processing unit is specifically configured to: detect a tissue associated with the abnormal data; determine that the location attribute of the tumor is an internal tumor if detecting that the tissue associated with the abnormal data only comprises the target organ; and determine that the location attribute of the tumor is an external tumor if detecting that the tissue associated with the abnormal data comprises the target organ and the blood vessel.

12. The apparatus according to claim 10, **characterized in that** the attribute information comprises a type attribute; in the aspect of the determining of the attribute information of the tumor of the target user according to the abnormal data, the processing unit is specifically configured to: determine boundary abnormal data in the abnormal data; determine the boundary characteristics of the tumor according to the boundary abnormality data; determine that the type attribute of the tumor of the target user is benign tumor if determining that the boundary characteristics are non-smooth continuous characteristics; and determine that the type attribute of the tumor of the target user is malignant tumor if detecting that the boundary characteristics are non-smooth continuous characteristics.

13. The apparatus according to claim 10, **characterized in that** the attribute information comprises a size attribute; in the aspect of the determining of the attribute information of the tumor of the target user according to the abnormal data, the processing unit is specifically configured to: determine spatial coordinate information of each abnormal data in the constant data; and determine the size attribute of the tumor of the target user according to the spatial coordinate information of each abnormal data.

14. The apparatus according to any one of claims 10-13, **characterized in that** in the aspect of the outputting of the attribute information of the tumor, the communication unit is specifically configured to: output the attribute information at a preset position of a display screen displaying an image of the tumor when a selection operation for a position of the tumor is detected.

15. The apparatus according to any one of claims 10-14, **characterized in that** in the aspect of the generating of the target medical image data according to the BMP data source, the processing unit is specifically configured to: introduce the BMP data source into a preset VRDS medical network model to obtain a first medical image data, wherein the first medical image data comprises the data set of the target organ and the data set of the blood vessel, and the data set of the blood vessel comprises the fusion data of the intersection position of the artery and the vein; introduce the first medical image data into a preset cross blood vessel network model to obtain a second medical image data, wherein the second medical image data comprises the data set of the target organ, the data set of the artery and the data set of the vein, and the first data in the data set of the artery and the second data in the data set of the vein are independent from each other; execute a first preset processing on the second medical image data to obtain target medical image data, wherein the first preset processing comprises at least one of the following operations: 2D boundary optimization processing, 3D boundary optimization processing and data enhancement processing, and the target medical image data comprises the data set of the target organ, the data set of the artery and the data set of the vein.

16. The apparatus according to claim 15, **characterized in that** in the aspect of the preforming of the 4D medical imaging according to the target medical image data, the communication unit is specifically configured to: screen enhanced data with a quality score greater than a preset score from the target medical image data as VRDS 4D imaging data.

17. The apparatus according to claim 10, **characterized in that** in the aspect of the determining of the bitmap BMP data source according to a plurality of scanned images associated with the target organ of the target user, the processing unit is specifically configured to: acquire a plurality of scanned images collected by a medical device and reflecting internal structure features of human body of the target user; screen at least one scanned image including the target organ from the plurality of scanned images, and take the at least one scanned image as medical digital imaging and communication DICOM data of the target user; parse the DICOM data to generate a image source of the target user, wherein the image source comprises Texture 2D/3D image volume data; execute a second preset process for the image source to obtain the BMP data source, wherein the second preset process comprises at least one of the following operations: VRDS limited contrast adaptive histogram equalization, mixed partial differential denoising and VRDS AI elastic deformation processing.

18. The apparatus according to claim 10, **characterized in that** in the aspect of the determining of abnormal data in the target medical image data, the processing unit is specifically configured to: compare the target medical image data with a pre-stored gray value template of the target tissue to obtain abnormal data with an unmatched comparison result, wherein the target tissue comprises the target organ and the blood vessel.

19. A medical imaging apparatus, **characterized in that** the apparatus comprises a processor, a memory, a communication interface, and one or more programs, the one or more programs are stored in the memory and configured to be executed by the processor, and the programs comprise instructions for executing the steps in the method according to any one of claims 1-9.

20. A computer-readable storage medium, **characterized in that** the computer-readable storage medium stores a computer program for electronic data exchange, and wherein the computer program causes a computer to execute the method according to any one of claims 1-9.

100

120

111

112

110

Fig. 1

Medical imaging apparatus determining a bitmap BMP data source according to a plurality of scanned images associated with a target organ of a target user — S201

The medical imaging apparatus generates target medical image data according to the BMP data source — S202

The medical imaging apparatus determines the abnormal data in the target medical image data — S203

The medical imaging apparatus determines attribute information of a tumor of the target user according to the abnormal data — S204

The medical imaging apparatus performs a 4D medical imaging according to the target medical image data, and outputting the attribute information of the tumor — S205

Fig. 2

Medical imaging apparatus
300

Application
processor 310

Communication
interface 330

Memory 320

One or more programs 321

Fig. 3

400

An apparatus for Processing of
Tumor Based on VRDS 4D
Medical Images

403

Storage unit

401

Processing unit

402

Communication unit

Fig. 4

## INTERNATIONAL SEARCH REPORT

| International application No. |
|---|
| **PCT/CN2019/101156** |

**A. CLASSIFICATION OF SUBJECT MATTER**

G16H 30/20(2018.01)i; G16H 50/20(2018.01)i; G06T 7/00(2017.01)i

According to International Patent Classification (IPC) or to both national classification and IPC

**B. FIELDS SEARCHED**

Minimum documentation searched (classification system followed by classification symbols)

G16H; A61B; G06T

Documentation searched other than minimum documentation to the extent that such documents are included in the fields searched

Electronic data base consulted during the international search (name of data base and, where practicable, search terms used)

WPI, EPODOC, CNPAT, CNKI, IEEE: 影像, 器官, 肿瘤, 病灶, 血管, 静脉, 动脉, 模型, image, dicom, organ, cancer, tumor, vessel, model

**C. DOCUMENTS CONSIDERED TO BE RELEVANT**

| Category* | Citation of document, with indication, where appropriate, of the relevant passages | Relevant to claim No. |
|---|---|---|
| Y | CN 109157284 A (GUNAGZHOU DIKA VISION TECHNOLOGY CO., LTD.) 08 January 2019 (2019-01-08) description, paragraphs [0050]-[0079], and figure 1 | 1-20 |
| Y | CN 108877922 A (SHEN, Yuanyao) 23 November 2018 (2018-11-23) description, paragraphs [0043]-[0044], and figures 3 and 4 | 1-20 |
| A | CN 108399942 A (QINGDAO HISENSE MEDICAL EQUIPMENT CO., LTD.) 14 August 2018 (2018-08-14) entire document | 1-20 |
| A | CN 108573490 A (WANG, Chengyan et al.) 25 September 2018 (2018-09-25) entire document | 1-20 |
| A | CN 109166105 A (NANJING GENERAL HOSPITAL OF NANJING MILITARY COMMAND OF CHINESE PLA) 08 January 2019 (2019-01-08) entire document | 1-20 |
| A | WO 2013098721 A2 (KONINKLIJKE PHILIPS ELECTRONICS N.V.) 04 July 2013 (2013-07-04) entire document | 1-20 |

☐ Further documents are listed in the continuation of Box C.    ☑ See patent family annex.

| * Special categories of cited documents: | |
|---|---|
| "A" document defining the general state of the art which is not considered to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **24 October 2019** | **20 November 2019** |

| Name and mailing address of the ISA/CN | Authorized officer |
|---|---|
| **China National Intellectual Property Administration** **No. 6, Xitucheng Road, Jimenqiao Haidian District, Beijing 100088** **China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2019/101156**

| Patent document cited in search report | | | Publication date (day/month/year) | Patent family member(s) | | | Publication date (day/month/year) |
|---|---|---|---|---|---|---|---|
| CN | 109157284 | A | 08 January 2019 | None | | | |
| CN | 108877922 | A | 23 November 2018 | None | | | |
| CN | 108399942 | A | 14 August 2018 | None | | | |
| CN | 108573490 | A | 25 September 2018 | None | | | |
| CN | 109166105 | A | 08 January 2019 | None | | | |
| WO | 2013098721 | A2 | 04 July 2013 | US | 2014365244 | A1 | 11 December 2014 |
| | | | | CN | 104025100 | A | 03 September 2014 |
| | | | | BR | 112014015758 | A2 | 04 July 2017 |
| | | | | EP | 2798549 | A2 | 05 November 2014 |
| | | | | JP | 2015510623 | A | 09 April 2015 |

Form PCT/ISA/210 (patent family annex) (January 2015)